# EUROPEAN PATENT APPLICATION

(11) **EP 3 214 532 A1**
(43) Date of publication of application: **06.09.2017**
(21) Application number: 14905024.7
(22) Date of filing: 25.12.2014
(51) Int. Cl.: G06F 3/044

(54) **MOBILE TERMINAL**

(30) Priority: 27.10.2014 CN 201410583515
(71) Applicant: Shenzhen Huiding Technology Co., Ltd., Shenzhen, Guangdong 518000 (CN)
(72) Inventor: LING, Wei, Shenzhen Guangdong 518000 (CN); DENG, Gengchun, Shenzhen Guangdong 518000 (CN)
(74) Representative: Valea AB
(86) International application number: PCT/CN2014/095029
(87) International publication number: WO 2016/065715

(57) **Abstract**

The present invention discloses a mobile terminal, including a capacitive touch screen, wherein the capacitive touch screen includes a touch cover plate and a sensing identifying module, the touch cover plate covering the sensing identifying module and having a thickness of 0.40 mm to 2.0 mm. In the embodiments of the present invention, the sensing identifying module is integrated into a touch screen, and a full touch panel is used. In this way, the problem of sense of difference which is brought by the independently assembled sensing identifying module is solved from the visual and tactile perspectives, the manufacturing procedure is simplified, production and utilization efficiencies are improved, and user's satisfaction is enhanced.

## Description

### TECHNICAL FIELD

The present invention relates to the technical field of mobile terminals, and in particular, relates to a mobile terminal with a touch screen equipped with a sensing identifying module.

### BACKGROUND

At present, a press-type capacitive sensing identifying module of the latest generation of smart handheld devices at home and abroad is embedded into a slot opened on a touch screen such that the sensing identifying module is exposed to a user's fingers for touches. Such design not only increases manufacturing difficulty of the touch screen, but also enhances sense of difference which is brought by the independently assembled sensing identifying module from the visual and tactile perspectives. As a result, the entire structure, appearance and user experience of a device are affected.

### SUMMARY

The present invention is intended to solve at least one of the above problems in the related art.

To this end, one objective of the present invention is to provide a mobile terminal, where a sensing identifying module is integrated into a touch screen thereof. With this technical solution, the problem of sense of difference which is brought by the independently assembled sensing identifying module is solved from the visual and tactile perspectives, the manufacturing procedure is simplified, production and utilization efficiencies are improved, and user's satisfaction of is bettered.

The present invention is implemented by: a mobile terminal, including a capacitive touch screen, wherein the capacitive touch screen includes a touch cover plate and a sensing identifying module, the touch cover plate covering the sensing identifying module and having a thickness of 0.40 mm to 2.0 mm.

The touch cover plate includes a first part and a second part formed by partially outwardly recessing a surface of the touch cover plate, the second part entirely or partially covering the sensing identifying module, the first part having a thickness of 0.40 mm to 2.0 mm, and the second part having a thickness of 0.2 mm to 0.7 mm.

In one embodiment, the second part is specifically a plane or sphere formed by partially downwardly recessing an upper surface of the touch cover plate.

In another embodiment, the second part is specifically a plane or sphere formed by partially upwardly recessing a lower surface of the touch cover plate, the sensing identifying module being entirely or partially located inside of the second part.

The upper surface of the touch cover plate corresponding to the second part is also partially downwardly recessed to form a plane or sphere.

In one embodiment, the touch screen further includes a touch sensor, the touch sensor being located beneath the touch cover plate.

A through hole is arranged between an upper surface and a lower surface of the touch sensor, the sensing identifying module being placed in the through hole, and being laminated to the touch cover plate.

The touch sensor is placed, side-by-side with the sensing identifying module, beneath the touch cover plate. In one embodiment, the sensing identifying module is one or more of a fingerprint identifying module, a heartbeat identifying module and a blood oxygen identifying module.

The additional aspects and advantages of the present invention are partially illustrated in the following description, and the other portions would become more obvious from the following description or would be known from the practice of the present invention.

### BRIEF DESCRIPTION OF THE DRAWINGS

FIG. 1 is a schematic three-dimensional exploded view of a mobile terminal according to an embodiment of the present invention;
FIG. 2 is a schematic section view of the mobile terminal as illustrated in FIG. 1;
FIG. 3 is a schematic section view of the mobile terminal as illustrated in FIG. 3 (a) and FIG. 3 (b);
FIG. 4 (a) and FIG. 4 (b) are schematic section views of the mobile terminal as illustrated in FIG. 1;
FIG. 5 is another schematic three-dimensional exploded view of the mobile terminal according to an embodiment of the present invention;
FIG. 6 (a) is a schematic section view of the mobile terminal as illustrated in FIG. 5;
FIG. 6 (b) is another schematic section view of the mobile terminal as illustrated in FIG. 5; and
FIG. 6 (c) is another schematic section view of the mobile terminal as illustrated in FIG. 5.

### DETAILED DESCRIPTION

The embodiments of the present invention are described in detail hereinafter. Examples of the described embodiments are given in the accompanying drawings, wherein the identical or similar reference numerals constantly denote the identical or similar elements or elements having the identical or similar functions. The specific embodiments described with reference to the attached drawings are all exemplary, and are intended to illustrate and interpret the present invention, which shall not be construed as causing limitations to the present invention.

In the description of the present invention, it should be understood that the terms "thickness", "upper", "lower", "front", "rear", "left", "right", "vertical", "horizontal", "top", "bottom", "inner", "outer" and the like indicate orientations and position relationships which are based on the illustrations in the accompanying drawings, and these terms are merely for ease and brevity of the description, instead of indicating or implying that the devices or elements shall have a particular orientation and shall be structured and operated based on the particular orientation. Accordingly, these terms shall not be construed as limiting the present invention.

In the description of the present invention, it should be noted that unless otherwise specified and defined, the terms "mounted", "coupled", "connected" and "fixed" and derivative forms thereof shall be understood in a broad sense, which, for example, may be understood as fixed connection, detachable connection or integral connection; may be understood as mechanical connection or electrical connection, or understood as direct connection, indirect connection via an intermediate medium, or communication between the interiors of two elements or interactions between two elements. Persons of ordinary skill in the art may understand the specific meanings of the above terms in the present invention according to the actual circumstances and contexts.

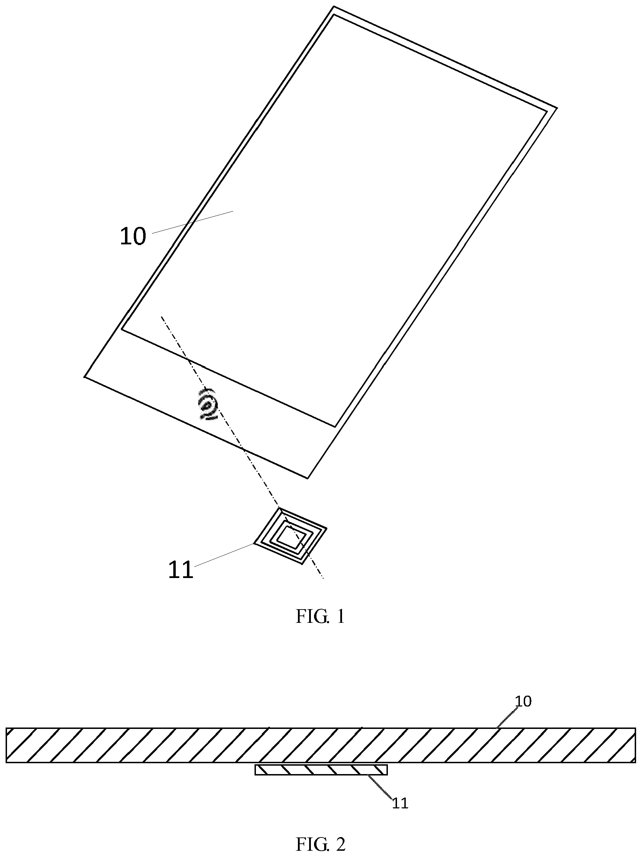

As illustrated in FIG. 1 and FIG. 2, this embodiment discloses a mobile terminal, including a touch screen. The touch screen includes a touch cover plate 10 and a sensing identifying module 11. The touch cover plate 10 covers the sensing identifying module 11. The touch cover plate has a thickness of 0.40 mm to 2.0 mm. Preferably, the sensing identifying module 11 is one or more of a fingerprint identifying module, a heartbeat identifying module and a blood oxygen identifying module.

Several embodiments of the touch cover plate are specifically described hereinafter (a sphere is used as an example in the drawings).

### Embodiment 1

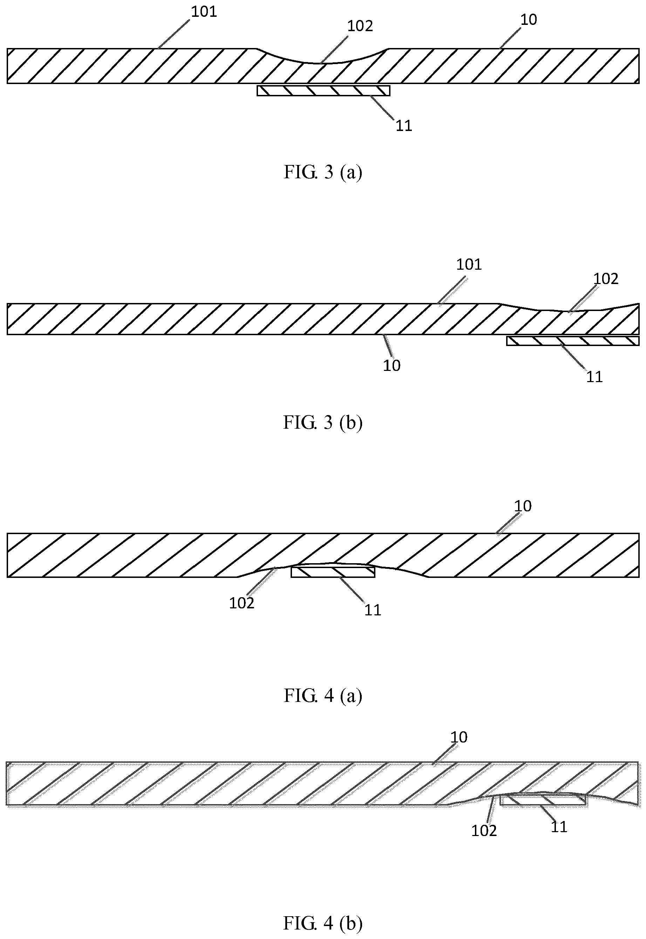

As illustrated in FIG. 3 (a) and FIG. 3 (b), the touch cover plate 10 includes a first part 101 and a second part 102 formed by partially outwardly recessing a surface of the touch cover plate, wherein the second part 102 entirely or partially covers the sensing identifying module 11. The second part 102 may be a plane or sphere (the sphere is only illustrated in the drawings). Preferably, in this embodiment, the first part has a thickness of 0.40 mm to 0.2 mm, and the second part has a thickness of 0.2 mm to 0.7 mm. Such design is intended to reduce the thickness of the touch cover late 10 covering the sensing identifying module 11, enhance the signal strength, and facilitate data collection by the sensing identifying module 11, and meanwhile indicate the location of the sensing identifying module, thereby facilitating user's operations.

### Embodiment 2

As illustrated in FIG. 4 (a) and FIG. 4 (b), the second part 102 of the touch cover plate 10 may also be a sphere formed by partially upwardly recessing a lower surface of the touch cover plate 10. The part except the sphere is the first part 101, and the sensing identifying module 11 is partially or entirely received inside the second part 102. In this embodiment, the upper surface of the touch cover plate corresponding to the second part is also partially downwardly recessed to form a plane or sphere (which is not illustrated in the drawings).

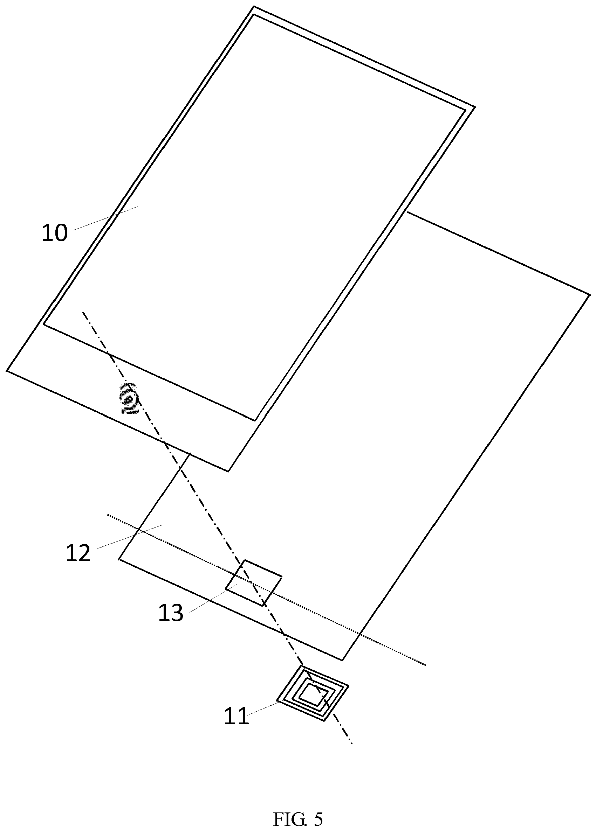

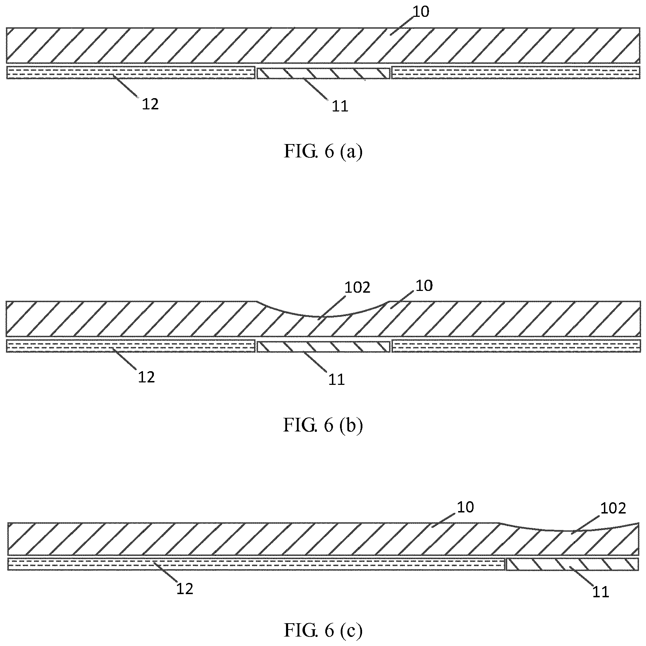

As illustrated in FIG. 5, the touch screen further includes a touch sensor 12, wherein the touch sensor 12 is located beneath the touch cover plate 10. As illustrated in FIG. 6 (a) and FIG. 6 (b), preferably, a through hole 13 may be arranged between an upper surface and a lower surface of the touch sensor 12, and the sensing identifying module 11 may be placed in the through hole and laminated to the touch cover plate 10. Nevertheless, as illustrated in FIG. 6 (c), the sensor 12 may be further placed, side-by-side with the sensing identifying module 11, beneath the touch cover plate 10. The location of the sensing identifying module 11 is not specifically defined. For example, the sensing identifying module 11 may be arranged at the location of a control board close to a physical HOME key or a virtual HOME key.

In the embodiments of the present invention, the sensing identifying module 11 is integrated into a touch screen assembly, and a full touch panel is used. In this way, the problem of sense of difference which is brought by the independently assembled sensing identifying module 11 is solved from the visual and tactile perspectives, the manufacturing procedure is simplified, production and utilization efficiencies are improved, and user's satisfaction is enhanced.

It should be noted that the mobile terminal herein may be a mobile phone, a tablet computer, or any device having a touch function.

Although the above description illustrates the embodiments of the present invention, it can be understood that the embodiments are merely exemplary, and shall not be construed as limitations to the present invention. Persons of ordinary skill in the art may derive variations, modifications, and replacements to the above embodiments within the scope of the present invention.

### Industrial Applicability

In the present invention, the sensing identifying module is integrated into a touch screen. In this way, the problem of sense of difference which is brought by the independently assembled sensing identifying module is solved from the visual and tactile perspectives, the manufacturing procedure is simplified, production and utilization efficiencies are improved, and user's satisfaction is enhanced.

## Claims

1. A mobile terminal, comprising a capacitive touch screen, wherein the capacitive touch screen comprises a touch cover plate and a sensing identifying module, the touch cover plate covering the sensing identifying module and having a thickness of 0.40 mm to 2.0 mm.

2. The mobile terminal according to claim 1, wherein the touch cover plate comprises a first part and a second part formed by partially outwardly recessing a surface of the touch cover plate, the second part entirely or partially covering the sensing identifying module, the first part having a thickness of 0.40 mm to 2.0 mm, and the second part having a thickness of 0.2 mm to 0.7 mm.

3. The mobile terminal according to claim 1, wherein the second part is specifically a plane or sphere formed by partially downwardly recessing an upper surface of the touch cover plate.

4. The mobile terminal according to claim 1, wherein the second part is specifically a plane or sphere formed by partially upwardly recessing a lower surface of the touch cover plate, the sensing identifying module being entirely or partially located inside the second part.

5. The mobile terminal according to claim 4, wherein the upper surface of the touch cover plate corresponding to the second part is also partially downwardly recessed to form a plane or sphere.

6. The mobile terminal according to claim 1, wherein the touch screen further comprises a touch sensor, the touch sensor being located beneath the touch cover plate.

7. The mobile terminal according to claim 6, wherein a through hole is arranged between an upper surface and a lower surface of the touch sensor, the sensing identifying module being placed in the through hole and being laminated to the touch cover plate.

8. The mobile terminal according to claim 6, wherein the touch sensor is placed, side-by-side with the sensing identifying module, beneath the touch cover plate.

9. The mobile terminal according to claim 1, wherein the sensing identifying module is one or more of a fingerprint identifying module, a heartbeat identifying module and a blood oxygen identifying module.
